# EUROPÄISCHE PATENTSCHRIFT

(11) **EP 2 055 269 B1**
(45) Veröffentlichungstag und Bekanntmachung des Hinweises auf die Patenterteilung: **18.04.2012**
(21) Anmeldenummer: 08165666.2
(22) Anmeldetag: 02.10.2008
(51) Int. Cl.: A61F 2/30, A61F 2/44

(54) **Höhenverstellbares Wirbelsäulenimplantat**
Height-adjustable spinal implant
Implant de colonne vertébrale réglable en hauteur

(30) Priorität: 30.10.2007 DE 102007052042
(43) Veröffentlichungstag der Anmeldung: 06.05.2009
(73) Patentinhaber: Kraus, Kilian, 97440 Werneck (DE)
(72) Erfinder: Kraus, Kilian, 97440 Werneck (DE)
(74) Vertreter: Mörtel & Höfner

(56) Entgegenhaltungen:
- WO-A-2004/052245
- WO-A-2005/055887
- US-A1- 2003 045 877
- US-A1- 2004 162 618

## Beschreibung

Die Erfindung betrifft ein höhenverstellbares Wirbelsäulenimplantat zum Einsetzen zwischen Wirbelkörper. Dieses Implantat umfasst ein erstes und ein zweites Bauteil, die längs einer Mittellängsachse des Implantates gegeneinander teleskopartig verschiebbar sind. Die Bauteile sind axial beweglich, jedoch gegeneinander drehfest aneinander gehalten. Jedes der Bauteile umfasst zumindest zwei an einer Basis fixierte Wandsegmente, welche sich sowohl in Richtung der Mittellängsachse als auch in Umfangsrichtung erstrecken. Die Wandsegmente weisen dabei einen Radialabstand zur Mittellängsachse auf. In Umfangsrichtung benachbarte Wandsegmente flankieren jeweils einen Zwischenraum, in den sich ein Wandsegment des jeweils anderen Bauteils erstreckt, und darin axial geführt ist. In einem von dem ersten und zweiten Bauteil umschlossenen Innenraum befindet sich ein Antriebselement, welches an dem ersten Bauteil gehalten ist, und mit dem das zweite Bauteil gegenüber dem ersten in axialer Richtung bewegbar ist. Ein derartiges höhenverstellbares Wirbelsäulenimplantat geht beispielsweise aus der WO 2005/055887 A2 hervor.

Ein solches Wirbelsäulenimplantat weist ein Antriebselement mit einer radial umlaufenden Nut auf. An den Freienden der Wandsegmente des ersten Bauteils sind radial in diese Nut eingreifende Vorsprünge vorhanden, mit denen das Antriebselement gehalten ist. Bei hohen mechanischen Belastungen des Wirbelsäulenimplantates besteht jedoch die Gefahr, dass in Folge einer Verbiegung der Wandsegmente oder der Vorsprünge das Antriebselement aus seiner Halterung gedrückt wird.

Aufgabe der vorliegenden Erfindung ist es, ein höhenverstellbares Wirbelsäulenimplantat anzugeben, welches hinsichtlich seiner mechanischen Belastbarkeit verbessert ist.

Die vorgenannte Aufgabe wird durch ein Wirbelsäulenimplantat nach Anspruch 1 gelöst.

Ein solches erfindungsgemäßes, höhenverstellbares Wirbelsäulenimplantat weist ein erstes und ein zweites Bauteil auf, welche längs einer Mittellängsachse des Implantates axial beweglich und gegeneinander drehfest aneinander gehalten sind. Das erste und zweite Bauteil weist jeweils zumindest zwei an einer Basis fixierte Wandsegmente auf, die sich zum einen in Richtung der Mittellängsachse und zum anderen, in einem Radialabstand von der Mittellängsachse, in Umfangsrichtung des Implantates erstrecken. Benachbarte Wandsegmente flankieren einen Zwischenraum, in den sich jeweils ein Wandsegment des anderen Bauteils erstreckt, und darin axial geführt ist. Das höhenverstellbare Wirbelsäulenimplantat weist außerdem ein Antriebselement auf, welches in einem von den Wandsegmenten umschlossenen Innenraum des Implantates angeordnet ist. Das zweite Bauteil wirkt mit dem Antriebselement nach der Art eines Schraubengetriebes zusammen.

Unter einem Schraubengetriebe ist in diesem Zusammenhang ein mechanisches Getriebe zu verstehen, bei dem eine Spindel und eine Antriebsmutter zusammenwirken. Die Spindel kann, von der Antriebsmutter angetrieben, in ihrer Längsrichtung bewegt werden. Für den Begriff des Schraubengetriebes ist es in diesem Zusammenhang irrelevant, ob die Spindel ein Innengewinde und die antreibende Mutter ein Außengewinde aufweist, oder ob die Spindel ein Außengewinde und die antreibende Mutter ein Innengewinde aufweist. Das Antriebselement, welches mit dem zweiten Bauteil nach der Art eines Schraubengetriebes zusammen wirkt, kann also sowohl ein Außengewinde als auch ein Innengewinde aufweisen.

Weiterhin erfindungsgemäß weist das Antriebselement einen koaxial zur Mittellängsachse des Wirbelsäulenimplantates angeordneten, zur Drehbetätigung des Antriebselements dienenden Zahnkranz auf. Das Antriebselement ist an dem ersten Bauteil in Lastrichtung abgestützt. Ein Wandsegment des ersten Bauteils ist von einer Zugriffsöffnung durchsetzt, über die der Zahnkranz zur Drehbetätigung des Antriebselementes mit Hilfe eines Handhabungswerkzeugs zugänglich ist. In dem Innenraum des Wirbelsäulenimplantates ist ein Halteelement vorhanden, welches die Freienden der Wandsegmente des ersten Bauteils miteinander verbindet.

Wird ein solches erfindungsgemäßes Wirbelsäulenimplantat einer hohen axialen Belastungen ausgesetzt, also mit einer Kraft beaufschlagt, die das erste und zweite Bauteil zusammendrückt, so tritt an der Stelle, an der das Antriebselement mit dem ersten Bauteil verbunden ist, eine hohe mechanische Belastung auf. Durch das in dem Innenraum des Implantates angeordnete Halteelement werden vorteilhaft die Freienden der Wandsegmente des ersten Bauteils stabilisiert. Insbesondere kann ein in radialer Richtung auf die Wandsegmente wirkendes Spreizmoment abgefangen werden. Die Belastbarkeit des Implantates kann signifikant erhöht werden.

Vorteilhafte Weiterbildungen des erfindungsgemäßen Wirbelsäulenimplantates gehen aus den im Folgendem angesprochenen Unteransprüchen hervor. Dabei kann das erfindungsgemäße Wirbelsäulenimplantat mit den Merkmalen eines oder mehrerer Unteransprüche vorteilhaft kombiniert werden.

Gemäß einer Ausführungsform des Wirbelsäulenimplantates sind das Halteelement und das erste Bauteil einstückig ausgebildet. Durch diese Maßnahme kann die Anzahl der Bauteile für das Wirbelsäulenimplantat reduziert werden, was fertigungstechnische Vorteile birgt. Außerdem kann die Belastbarkeit des Wirbelsäulenimplantates durch die einteilige Konstruktion erhöht werden.

Gemäß einer weiteren vorteilhaften Ausführungsform ist das Antriebselement an dem Halteelement um die Mittellängsachse drehbar fixiert, wobei das Antriebselement sich vorzugsweise in Lastrichtung an einer rechtwinklig zur Mittellängsachse des Halteelements verlaufenden Stützfläche abstützt. Bei einer solchen vorteilhaften Ausführungsform erfüllt das Halteelement eine Doppelfunktion. Zum einen werden die Wandsegmente durch das Halteelement stabilisiert, zum anderen bietet das Halteelement dem Antriebselement eine stabile Stützfläche.

Das Antriebselement kann, gemäß einer vorteilhaften Weiterbildung, mit dem Zahnkranz auf der Stützfläche abgestützt sein. Die Kontaktfläche zwischen dem Zahnkranz und der Stützfläche kann klein gewählt werden, so dass lediglich eine geringe Reibung zwischen diesen Bauteilen auftritt. Beispielsweise kann der Zahnkranz mit den Spitzen seiner Zähne auf der Stützfläche aufstehen. Besonders vorteilhaft kann das Verletzungsrisiko für umliegendes Gewebe dadurch verringert werden, dass der Zahnkranz mit der Stützfläche in Kontakt steht. Zumindest aus dieser Richtung kann, bedingt durch die Stützfläche, das Eindringen von Gewebe in das Schraubengetriebe verhindert werden. Ein solcher Schutz des Schraubengetriebes vor möglicherweise aus der Richtung der Stützfläche in den Zahnkranz eindringendem Gewebe ist besonders vorteilhaft, da der Innenraum des Wirbelsäulenimplantates üblicherweise mit Knochenspänen oder einem Knochenersatzmaterial gefüllt wird.

Eine weitere Verbesserung zum Schutz des umliegenden Gewebes wird gemäß einer weiteren Ausgestaltung des Wirbelsäulenimplantates dadurch erreicht, dass der Zahnkranz nach der Art eines Kronenrades eines Kronenradgetriebes ausgestaltet ist, wobei das Halteelement eine die Stützfläche durchbrechende Ausnehmung aufweist, die mit einer Zugriffsöffnung in Verbindung steht. Besonders vorteilhaft erfolgt gemäß dieser Ausführungsform der Antrieb des Kronenrades von der Seite der Stützfläche her. Bei einem solchen Wirbelsäulenimplantat kann der Eintritt von Gewebe in den Bereich des Schraubengetriebes, bedingt durch den auf der Stützfläche aufstehenden Zahnkranz, weitgehend vermieden werden. Außerdem erfolgt der Antrieb des Kronenrades mit einem Handhabungswerkzeug, das durch die Zugriffsöffnung in dem Wandsegment auf das Kronenrad zugreift. Im Bereich der Wandsegmente an dem Implantat anliegendes Gewebe kann weitgehend von dem Schraubengetriebe ferngehalten werden, da lediglich in dem Bereich der Zugriffsöffnung die Möglichkeit besteht, dass Gewebe mit dem Schraubengetriebe in Kontakt kommt. Die Zugriffsöffnung ist jedoch überwiegend durch das Handhabungswerkzeug verdeckt. So kann auch in diesem Bereich das Verletzungsrisiko für das umliegende Gewebe verringert werden.

Die Zugriffsöffnung ist bevorzugt im Bereich des Halteelementes in das Wandsegment eingebracht. Eine Zugriffsöffnung stellt gegebenenfalls eine Schwächung des betreffenden Wandsegmentes dar. Wird vorteilhafterweise die Zugriffsöffnung im Bereich des Halteelementes in das betreffende Wandsegment eingebracht, so befindet sich diese Zugriffsöffnung in einem mechanisch stabilen Bereich des Wirbelsäulenimplantates. Die mechanische Schwächung des Wirbelsäulenimplantates durch die Zugriffsöffnung kann somit minimiert werden.

Nach einer weiteren Ausführungsform weist das Halteelement eine Durchgangsöffnung auf, die von einem Fixierelement durchgriffen ist. Ein Ende des Fixierelementes ist mit dem Antriebselement verbunden, das andere Ende weist ein Hintergriffselement auf, dass das Halteelement auf seiner dem Antriebselement abgewandten Seite hintergreift. Das Antriebselement ist folglich sowohl in Lastrichtung als auch entgegen der Lastrichtung mit dem Halteelement mechanisch verbunden. Somit ist das Wirbelsäulenimplantat sowohl auf Druck wie auch auf Zug belastbar, was seinen Einsatzbereich erweitert. Das Halteelement und das Fixierelement können zu ihrer mechanischen Verbindung beispielsweise miteinander verschraubt sein.

Das Antriebselement ist, gemäß einer Ausführungsform, derart ausgestaltet, dass dieses ein Außengewinde aufweist, welches mit einem Innengewinde des zweiten Bauteils kämmt. Ein solches Zusammenwirken zwischen dem Antriebselement und dem zweiten Bauteil stellt ein besonders einfaches und mechanisch belastbares Schraubengetriebe dar. Besonders vorteilhaft kann das Innengewinde in die Innenseiten der Wandsegmente des zweiten Bauteils eingelassen sein.

Gemäß einer weiteren Ausführungsform ist das Außengewinde an dem dem zweiten Bauteil zugewandten Ende des Antriebselementes angeordnet. Vorteilhaft wird das Antriebselement somit lediglich Druckbelastungen und keinen Zugbelastungen ausgesetzt.

Nach einer weiteren Ausführungsform, ist das Antriebselement derart an dem ersten Hülsenteil angeordnet, dass das Außengewinde, in Axialrichtung gesehen, sich außerhalb des ersten Hülsenteils befindet. Durch eine solche Anordnung des Antriebselementes an dem ersten Hülsenteil kann das zweite Hülsenteil gegenüber dem ersten besonders weit verfahren werden. Außerdem erlaubt die Lage des Antriebselementes den Außenradius des Außengewindes so groß zu wählen, dass dieser in den Bereich der Wandstärke des zweiten Bauteils hineinragt. Ein Innengewinde kann somit, besonders vorteilhaft, in die Innenwand des zweiten Bauteils gefräst werden.

Zur Verbesserung der Handhabbarkeit ist das Wirbelsäulenimplantat mit einem Handhabungswerkzeug verbindbar. Insbesondere ist es möglich, mit Hilfe des Handhabungswerkzeuges das Wirbelsäulenimplantat während eines medizinischen Eingriffes exakt in eine gewünschte Position zu positionieren. Zur Aufnahme des Wirbelsäulenimplantates durch ein Handhabungswerkzeug befindet sich in demjenigen Wandsegment, welches die Zugriffsöffnung aufweist, mindestens ein der Radialfixierung, d.h. der Fixierung des Wirbelsäulenimplantates gegenüber dem Handhabungswerkzeug in einer Richtung radial zur Mittellängsachse des Implantates, dienendes Hintergriffselement. Nach einer vorteilhaften Weiterbildung des Hintergriffselementes weist dieses eine in Richtung des Innenraums des Implantates zeigende Hintergriffsfläche auf. Eine derart orientierte Hintergriffsfläche gestattet eine einfache und effektive Aufnahme des Implantates unter radialer Fixierung durch das Handhabungswerkzeug.

Nach einer weiteren Ausführungsform, die auch für sich genommen schon eine Lösung der erfindungsgemäßen Aufgabe darstellt, weist das Wirbelsäulenimplantat die folgenden Merkmale auf:
Das erfindungsgemäße höhenverstellbare Wirbelsäulenimplantat weist ein erstes und ein zweites Bauteil auf, welche längs der Mittellängsachse des Implantates axial beweglich und gegeneinander drehfest aneinander gehalten sind. Das erste und zweite Bauteil weist jeweils zumindest zwei an einer Basis fixierte Wandsegmente auf, die sich zum einen in Richtung der Mittellängsachse und zum anderen in einem Radialabstand von der Mittellängsachse, in Umfangsrichtung des Implantates erstrecken. Benachbarte Wandsegmente flankieren jeweils einen Zwischenraum, in den sich jeweils ein Wandsegment des anderen Bauteils erstreckt, und darin axial geführt ist. Das höhenverstellbare Wirbelsäulenimplantat weist außerdem ein Antriebselement auf, welches in einem von den Wandsegmenten umschlossenen Innenraum des Implantates angeordnet ist. Das zweite Bauteil wirkt mit dem Antriebselement nach der Art eines Schraubengetriebes zusammen. Das Antriebselement umfasst einen koaxial zur Mittellängsachse des Wirbelsäulenimplantates angeordneten, zur Drehbetätigung des Antriebselements dienenden Zahnkranz. Das Antriebselement ist an dem ersten Bauteil in Lastrichtung abgestützt. Ein Wandsegment des ersten Bauteils ist von einer Zugriffsöffnung durchsetzt, über die der Zahnkranz zur Drehbetätigung des Antriebselementes mit Hilfe eines Handhabungswerkzeugs zugänglich ist. Die Wandsegmente des ersten und zweiten Bauteils sind durch einen in Radialrichtung wirksamen Formschluss miteinander verbunden.

Eine solche Ausführungsform stellt sowohl für sich allein genommen als auch in Verbindung mit einer der vorstehenden Ausführungsformen eine vorteilhafte Lösung der erfindungsgemäßen Aufgabe dar.

Bei einer hohen Belastungen des Wirbelsäulenimplantates, insbesondere bei einer Belastung in axialer Richtung, wirkt speziell auf das zweite Bauteil ein Spreizmoment. Zur Verbesserung der Stabilität des Wirbelsäulenimplantates können daher das erste und zweite Bauteil durch einen radial nach außen wirksamen Formschluss aneinander gehalten sein.

Zur Herstellung eines in Radialrichtung wirksamen Formschlusses sind, nach einer weiteren Ausführungsform, die in Umfangsrichtung einander zugewandten Schmalseiten der Wandsegmente zumindest auf einem Teil ihrer Länge derart ausgestaltet, dass diese formschlüssig ineinandergreifen. Ein Formschluss zwischen den Wandsegmenten des ersten und zweiten Bauteils ist besonders vorteilhaft, da sich die Wandsegmente in einer axialen Richtung des Wirbelsäulenimplantates erstrecken, und daher eine einfache Führung in Axialrichtung bei gleichzeitiger Stabilisierung in radialer Richtung erlauben.

Gemäß einer vorteilhaften Weiterbildung sind die Wandsegmente an ihren Schmalseiten jeweils mit einer in Umfangsrichtung vorspringenden Leiste versehen. Die Leisten des zweiten Bauteils hintergreifen dabei diejenigen des ersten Bauteils. Die vorgenannte Ausführungsform stellt eine besonders einfache Lösung für einen radial wirksamen Formschluss dar.

Alternativ zu einem radial nach außen wirksamen Formschluss zwischen den Wandsegmenten des ersten und zweiten Bauteils können, gemäß einer weiteren Ausführungsform, das Antriebselement und die Wandsegmente des zweiten Bauteils nach einem radial nach außen wirksamen Formschluss zusammenwirken. Demgemäß weist das Außengewinde des Antriebselementes und das Innengewinde des zweiten Bauteils, in eine radiale Richtung betrachtet, gegenüber der Mittellängsachse des Implantates geneigte Flanken auf. Dabei sind die dem zweiten Bauteil zugewandten, tragenden Flanken des Außengewindes des Antriebselementes abgeschrägt, und schließen mit der Mittellängsachse einen zu dem zweiten Bauteil hin geöffneten spitzen Winkel ein. Die mit den zuvor genannten Flanken des Antriebsteils zusammenwirkenden Gegenflanken des Innengewindes des zweiten Bauteils sind entsprechend komplementär ausgestaltet. Spezialfälle derart ausgestalteter Gewinde werden auch als Kompressionsgewinde oder negative Gewinde bezeichnet. Wirkt eine Kraft in axiale Richtung des Implantates, wird also das zweite Bauteil in Richtung des Antriebselementes gedrückt, so wirken die entsprechenden tragenden Flanken des zweiten Bauteils und des Antriebselementes derart zusammen, dass die Wandsegmente des zweiten Bauteils ein nach innen gerichtetes Kraftmoment erfahren. Bei einer Belastung des Implantates werden die Wandsegmente des zweiten Bauteils also in Richtung der Mittellängsachse nach innen gezogen. Dieses Moment wirkt einem Spreizmoment der Wandsegmente, welches nach außen gerichtet ist, entgegen. Die mechanische Belastbarkeit des Implantates kann auf diese Weise signifikant erhöht werden.

Gemäß einer Ausführungsform ist das Innengewinde des zweiten Bauteils sowie das Außengewindes des Antriebselementes, betrachtet in einer die Mittellängsachse enthaltenden Ebene, schwalbenschwanzförmig ausgestaltet. Durch eine derartige Ausgestaltung der ineinandergreifenden Gewinde kann deren Führung sowie die mechanische Stabilität des Wirbelsäulenimplantates weiter verbessert werden. Außerdem bietet ein solches schwalbenschwanzförmiges Gewinde sowohl für Druck- als auch für Zugbelastungen die Wirkung eines Kompressionsgewindes.

Ein Wirbelsäulenimplantat wird, nach einer Ausführungsform, zum Ersatz eines beschädigten Wirbelkörpers zwischen benachbarte Wirbelkörper unter Verwendung von stirnseitig vorhandenen Stützplatten eingesetzt. Diese Stützplatten erlauben durch ihre spezielle Ausgestaltung nicht nur eine verrutschsichere Verbindung mit dem benachbarten Wirbelkörpern, sondern erlauben es außerdem den Winkel zwischen den Oberflächen der benachbarten Wirbelkörper und der Mittellängsachse des Implantates flexibel einzustellen. Besonders vorteilhaft ist das Wirbelsäulenimplantat, gemäß einer Ausführungsform, zumindest teilweise aus einem Werkstoff auf der Basis von Edelstahl, Titan oder einem Polyetherketon (PEEK) gefertigt. Diese Werkstoffen sind medizinisch erprobt, weisen eine hohe Belastbarkeit auf, und sind daher für die Konstruktion von Wirbelsäulenimplantaten besonders geeignet.

Weitere vorteilhafte Ausgestaltungen des erfindungsgemäßen Wirbelsäulenimplantates gehen aus den vorstehend nicht angesprochenen Unteransprüchen sowie insbesondere aus der nachfolgend erläuterten Zeichnung hervor. Dabei zeigen deren
Fig. 1 ein Wirbelsäulenimplantat,
Fig. 2 und 3 ein erstes uns zweites Bauteil des Wirbelsäulenimplantats,
Fig. 4 ein Antriebselement,
Fig. 5 ein Fixierelement jeweils in Perspektivansicht,
Fig. 6 einen Längsschnitt durch das Wirbelsäulenimplantat,
Fig. 7 bis 9 Detailansichten im Bereich des Antriebselementes und
Fig. 10 bis 12 Detailansichten der Wandsegmente des ersten und zweiten Bauteils in Perspektivansicht sowie in einer Querschnittsansicht.

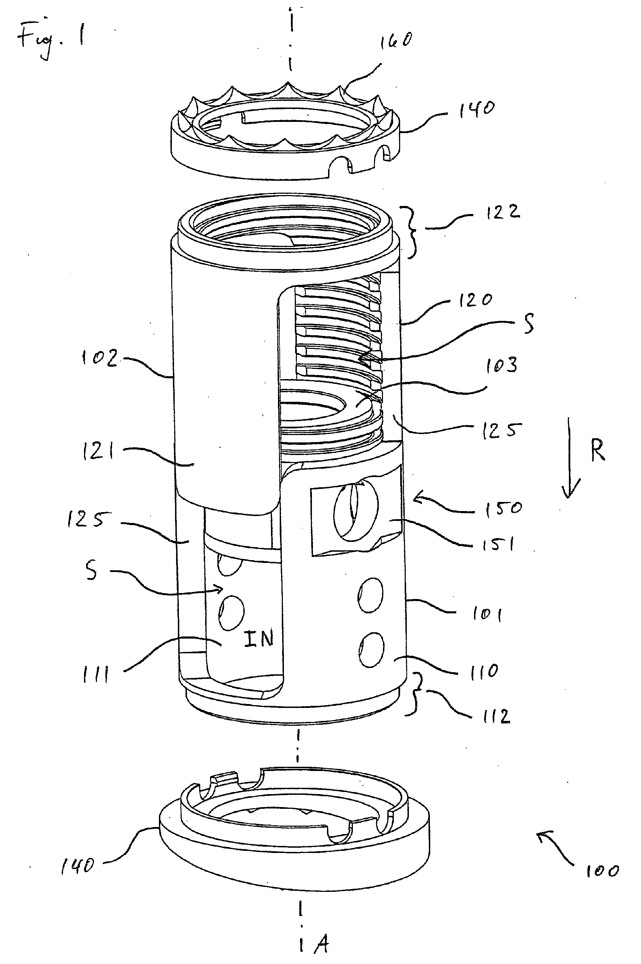

Fig. 1 zeigt ein Wirbelsäulenimplantat 100, mit einem ersten und zweiten Bauteil 101, 102, die koaxial zu einer Mittellängsachse A orientiert sind. Das erste und zweite Bauteil 101, 102 weist jeweils im Bereich einer Basis 112, 122 miteinander verbundene Wandsegmente 110, 111, 120, 121 auf. Die Wandsegmente 110, 111, 120, 121 flankieren - im Umfangsrichtung des Implantates 100 betrachtet - jeweils einen Zwischenraum S. Die Wandsegmente 110, 111 des ersten Bauteils 101 greifen dabei in die entsprechenden Zwischenräume S zwischen den Wandsegmenten 120, 121 des zweiten Bauteils 102. Umgekehrt greifen die Wandsegmente 120, 121 des zweiten Bauteils 102 in die zwischen den Wandsegmenten 110, 111 des ersten Bauteils 101 vorhandenen Zwischenräume S. Das erste und zweite Bauteil 101, 102 sind in Axialrichtung, also entlang der Mittellängsachse A, gegeneinander teleskopartig verschiebbar, in Radialrichtung jedoch drehfest aneinander gehalten. Die Wandsegmente 110, 111, 120, 121 des ersten und zweiten Bauteils 101, 102 weisen nach der Art von Segmenten einer Zylindermantelfläche zu der Mittellängsachse A einen radialen Abstand auf, und erstrecken sich sowohl in Umfangsrichtung als auch in Axialrichtung des Implantates 100. In Umfangsrichtung weisen die Wandsegmente 110, 111, 120, 121 einander zugewandte Schmalseiten 125 auf. Die jeweiligen Schmalseiten 125 des ersten und zweiten Bauteils 101, 102 gleiten bei axialer Bewegung der Bauteile 101, 102 aufeinander ab. In Umfangsrichtung betrachtet liegen die Schmalseiten 125 der Wandsegmente 110, 111, 120, 121 aneinander an, und verriegeln auf diese Weise das erste und zweite Bauteil 101, 102 drehfest gegeneinander.

An den Enden des ersten bzw. zweiten Bauteils 101, 102, im Bereich der jeweiligen Basis 112, 122, sind Stützplatten 140 mit dem Wirbelsäulenimplantat 100 verbindbar. Diese Stützplatten 140 unterstützen den anatomisch richtigen Sitz des Wirbelsäulenimplantates 100, sowie dessen Verankerung zwischen den gesunden Wirbelkörpern. Zur anatomischen Anpassung des Wirbelsäulenimplantates 100 kann, wie anhand der unteren in Fig. 1 gezeigten Stützplatte 140 ersichtlich, der Winkel zwischen derjenigen Oberfläche der Stützplatte 140, die mit dem benachbarten gesunden Wirbelkörper in Kontakt steht, und der Mittellängsachse A des Wirbelsäulenimplantates 100 von der Senkrechten abweichen. Um ein Verwachsen des Wirbelsäulenimplantates 100 mit dem umliegenden Gewebe zu erleichtern, und um einen verrutschsicheren Halt des Wirbelsäulenimplantates 100 zwischen den gesunden Wirbelkörpern zu gewährleisten, können die Stützplatten 140 mit Dornen oder Fortsätzen 160 versehen sein.

Während eines operativen Eingriffes ist es erforderlich das Wirbelsäulenimplantat 101 sowohl seiner Länge bzw. Höhe nach anzupassen, als auch zwischen den benachbarten Wirbelkörpern exakt zu positionieren. Die Anpassung der Höhe bzw. Länge des Wirbelsäulenimplantates 100 erfolgt durch die teleskopartige Verschiebung des ersten und zweiten Bauteils 101, 102. Zur Verschiebung des ersten und zweiten Bauteils 101, 102 gegeneinander befindet sich ein Antriebselement 103 in einem von den Bauteilen 101, 102 umschlossenen Innenraum IN. Der Innenraum IN ist dasjenige Volumen, welches in radialer Richtung zu der Mittellängsachse A des Wirbelsäulenimplantates 100 von den Wandsegmenten 110, 111, 120, 121 des ersten bzw. zweiten Bauteils 101, 102 begrenzt ist. In axialer Richtung wird der Innenraum IN von der jeweiligen Basis 112, 122 des ersten bzw. zweiten Bauteils 101, 102 begrenzt. Der Innenraum IN ist von den Enden des Wirbelsäulenimplantates 100 her zugänglich und erstreckt sich über dessen volle Länge. Knochenmaterial der benachbarten Wirbelkörper kann in das Wirbelsäulenimplantat 100 einwachsen, so dass im Laufe der Zeit die Wirbelkörper, durch den Innenraum IN hindurch, miteinander verwachsen.

Das Wirbelsäulenimplantat 100 ist im Wesentlichen Druckbelastungen ausgesetzt. Das mit dem zweiten Bauteil 102 nach der Art eines Schraubengetriebes zusammenwirkende Antriebselement 103 ist daher in Lastrichtung R an dem ersten Bauteil 101 abgestützt. Zur Drehbetätigung des Antriebselements 103 kann ein nicht dargestelltes Handhabungswerkzeug durch die Zugriffsöffnung 150 auf das Antriebselement 103 zugreifen. Das Handhabungswerkzeug kann außerdem zur exakten Positionierung des Wirbelsäulenimplantates 100 verwendet werden. Zu diesem Zweck kann das Handhabungswerkzeug in eine auf der Außenseite des eines Wandsegmentes 110 des ersten Bauteils 101 vorhandene Abplattung 151 eingreifen.

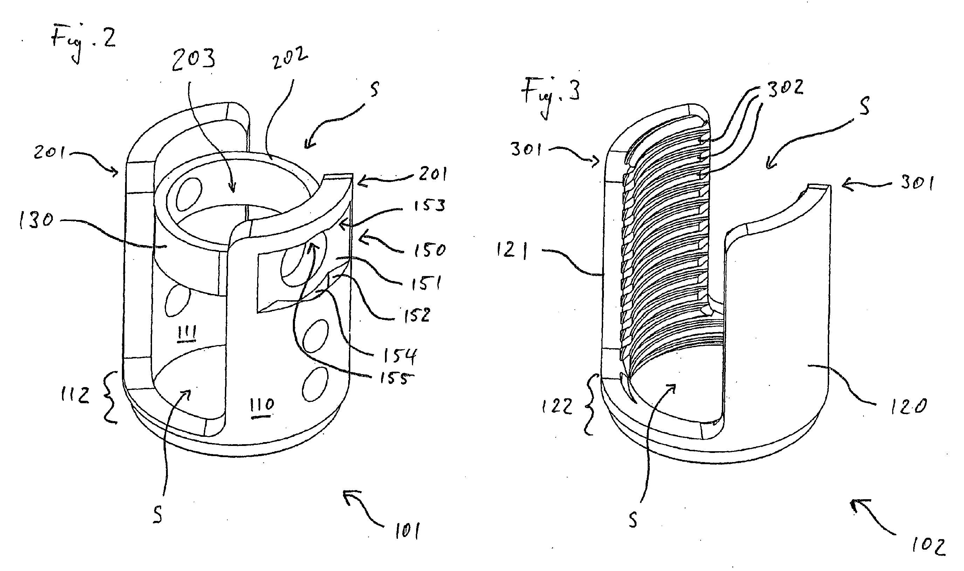

Für die weiteren Ausführungen wird neben auf Fig. 1 auf die Figuren 2 bis 4 verwiesen, in denen das erste Bauteil 101, das zweite Bauteil 202 und das Antriebselement 103 jeweils einzeln in Perspektivansichten dargestellt sind.

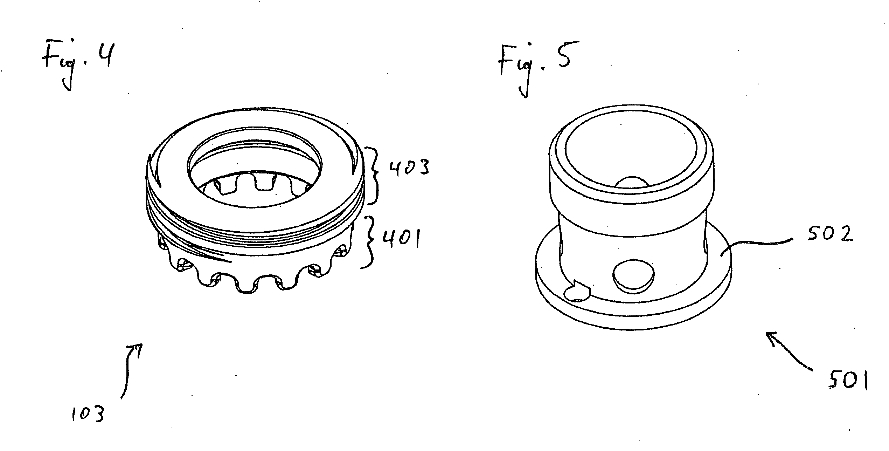

Fig. 4 zeigt ein Ausführungsbeispiel für das Antriebselement 103. Das Antriebselement 103 weist auf seiner dem ersten Bauteil 101 zugewandten Seite eine Zahnkranz 401 auf, der zum Antrieb des Antriebselementes 103 mit einem nicht dargestellten Ritzel nach der Art eines Kronenradgetriebes zusammenwirkt. An seiner radialen Außenfläche weist das Antriebselement 103 ein Außengewinde 403 auf, welches mit einem auf der Innenseite der Wandsegmente 120, 121 des zweiten Bauteils 102 vorhandenen Innengewinde 302 zusammen wirkt (vgl. Fig. 3).

Das Antriebselement 103 stützt sich in Lastrichtung R mit den Spitzen der Zähne des Zahnkranzes 401 auf einer Stützfläche 202 eines Halteelementes 130 ab (vgl. Fig. 2). Das Antriebselement 103 weist folglich lediglich eine geringe Kontaktfläche mit der Stützfläche 202 auf.

Das erste und zweite Bauteil 101, 102 des Wirbelsäulenimplantates 100 werden gegeneinander teleskopartig mit Hilfe des Antriebselementes 103 bewegt. Dabei kämmt das Außengewinde 403 des Antriebselementes 103 mit dem Innengewinde 302 des zweiten Bauteils 102. Durch eine Rotation des Antriebselementes 103 um die Mittellängsachse A wird das zweite Bauteil 102 von dem Antriebselement 103 nach der Art eines Schraubengetriebes angetrieben (vgl. Fig. 1).

Das Antriebselement 103 kann durch ein nicht dargestelltes Ritzel angetrieben werden, welches mit dem Zahnkranz 401 an der Unterseite des Antriebselementes 103 zusammenwirkt. Das das Antriebselement 103 antreibende Ritzel ist bevorzugt um eine radial zu der Mittellängsachse A orientierte weitere Achse drehbar, und wird durch eine Welle geführt. Ein derartiges Antriebsritzel kann durch die Zugriffsöffnung 150 in den Bereich des Zahnkranzes 401 des Antriebselementes 103 verbracht werden (vgl. Fig. 2). Ein Wandsegment 110 des ersten Bauteils 101 weist zu diesem Zweck die Zugriffsöffnung 150 auf.

Fig. 2 zeigt eine Perspektivansicht des ersten Bauteils 101. Die Freienden 201 der Wandsegmente 110, 111 des ersten Bauteils sind durch ein vorzugsweise ringförmiges Halteelement 130, welches insbesondere einstückig mit dem ersten Bauteil 101 ausgebildet sein kann, verbunden. Auf der in Fig. 2 nach oben weisenden Stützfläche 202 sitzt das in Fig. 4 gezeigte Antriebselement 103 mit seinem Zahnkranz 401 auf. Die Stützfläche 202 ist weiterhin vorzugsweise rechtwinklig zur der Mittellängsachse A orientiert.

Zur Drehbetätigung des Antriebselementes 103 mit dem erwähnten Ritzel greift das Handhabungswerkzeug durch die in dem Wandsegment 110 des ersten Bauteils 101 vorhandene Zugriffsöffnung 150 auf den Zahnkranz 401 des Antriebselementes 303 zu. Das zum Antrieb des Antriebselements 103 mit dem Zahnkranz 401 kämmende Ritzel befindet sich, durch eine Welle geführt, an der Spitze eines nicht dargestellten Handhabungswerkzeuges. Um den Zugriff des Ritzels auf den Zahnkranz 401 zu ermöglichen, weist die Stützfläche 202 eine Ausnehmung auf. Die ringförmig in Radialrichtung umlaufende Stützfläche 202 ist also im Bereich der Zugriffsöffnung 150 unterbrochen, so dass ein durch die Zugriffsöffnung 150 eingeführtes Ritzel des Handhabungswerkzeuges in der in Fig. 2 gezeigten Orientierung des ersten Bauelementes 101 von unten her auf das Antriebselement 103 bzw. dessen Zahnkranz 401 zugreifen kann. Das Ritzel des Handhabungswerkzeuges und der Zahnkranz 401 des Antriebselementes wirken dabei vorzugsweise nach der Art eines Kronenradgetriebes zusammen.

Während eines operativen Eingriffes ist es sowohl erforderlich das Wirbelsäulenimplantat 101 seiner Länge bzw. Höhe nach anzupassen, als auch es zwischen den benachbarten gesunden Wirbelkörpern exakt zu positionieren. Die Höhe bzw. Länge des Wirbelsäulenimplantates 100 wird durch die teleskopartige Verschiebung des ersten und zweiten Bauteils 101, 102 durch den zuvor beschriebenen Antrieb realisiert. Zur exakten Positionierbarkeit des Wirbelsäulenimplantates 100 wird dieses mit einem Handhabungswerkzeug verbunden. In diesem Zusammenhang ist zu berücksichtigen, dass die Verbindung zwischen dem Wirbelsäulenimplantat 100 und einem Handhabungswerkzeug drehfest und zuverlässig, aber auch problemlos und insbesondere ohne die zu Hilfenahme weiterer Werkzeuge lösbar sein soll. Das die Zugriffsöffnung 150 aufweisende Wandsegment 110 weist deshalb der Radialfixierung des Handhabungswerkzeug dienende Hintergriffselemente 154, 155 auf (vgl. Fig. 2). Die Hintergriffselemente 154, 155 sind in die sich senkrecht zur Mittellängsachse A erstreckenden Begrenzungsflächen 152, 153 eingelassen. Ein Handhabungswerkzeug, welches in die Abflachung 151 eingreift, kann somit durch die in Richtung des Innenraums IN des Implantates 100 weisenden Flächen der Hintergriffselemente 154, 155 das Wirbelsäulenimplantat 100 in Axialrichtung des Handhabungswerkzeuges verriegeln. Zur drehfesten Verriegelung des Wirbelsäulenimplantates 100 gegenüber der Längsachse des Handhabungswerkzeuges, können entsprechende Backen des Handhabungswerkzeuges mit den Begrenzungsflächen 152, 153 zusammen wirken. Die Axialrichtung des Handhabungswerkzeugs ist im Wesentlichen senkrecht, also radial zu der Mittenlängsachse A des Wirbelsäulenimplantates 100 orientiert.

Zur teleskopartigen Höhenverstellung des Wirbelsäulenimplantates 100 weist das Antriebselement 103 ein Außengewinde 403 an dem dem zweiten Bauteil 102 zugewandten Ende auf. Bei einer Druckbelastung des Wirbelsäulenimplantates 100, die vermittelt über das Innengewinde 302 des zweiten Bauteils 102 auf das Antriebselement 103 bzw. dessen Außengewinde 403 übertragen wird, wirken lediglich Druckkräfte auf das Antriebselement 103, die an die Stützfläche 203 des Halteelementes 130 weiter gegeben werden. Das Antriebselement 103 ist vorzugsweise so angeordnet, dass das Außengewinde 403 über die in Fig. 2 am oberen Ende der Freienden 201 des ersten Bauteils 101 sichtbaren Flächen hinaus ragt. Das Außengewinde 403 des Antriebselementes 103 erstreckt sich somit - in Radialrichtung betrachtet - über die Innenwand der Wandsegmente 110, 111 des ersten Bauteils 101 hinaus. Auf diese Weise ist es einfach möglich, dass das Außengewinde 403 des Antriebselementes 103 mit einem Innengewinde, welches direkt in die Wandsegmente 120, 121 des zweiten Bauteils 202 eingelassen ist, kämmt. Außerdem ermöglicht eine derartige Anordnung des Außengewindes 403 des Antriebselementes 103 einen besonders langen Verfahrweg des ersten und zweiten Bauelementes 101, 102 gegeneinander.

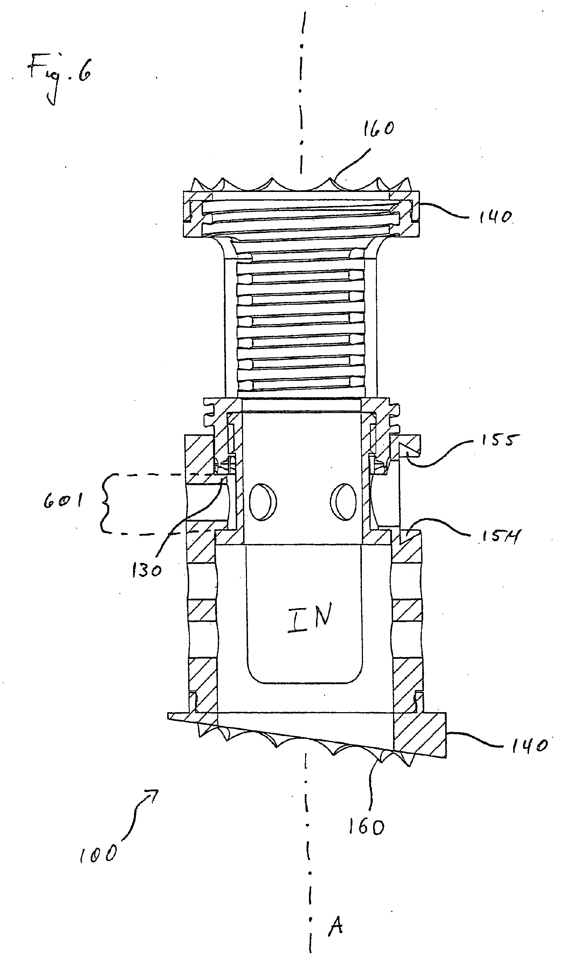

Die Stützfläche 202 des Halteelementes 130, des in Fig. 2 gezeigten ersten Bauteils 101, weist mittig eine Durchgangsöffnung 203 auf. Diese Durchgangsöffnung 203 ist von einem Fixierelement 501, welches in Fig. 5 in Perspektivansicht dargestellt ist, durchgriffen. Das Fixierelement 501 ist mit dem Antriebselement 103 beispielsweise durch eine Verschraubung fixiert. Das an dem in Fig. 5 dargestellten unteren Ende des Fixierelementes 501 vorhandene Hintergriffselement 502 hintergreift das Halteelement 130 auf der dem zweiten Bauteil 102 abgewandten Seite. Das Antriebselement 130 und das Fixierelement 501 bilden so ein gemeinsames Bauteil aus, das eine Nut 601 (vgl. Fig. 6) aufweist, in welcher das Halteelement 130 zu liegen kommt.

Fig. 6 zeigt einen Längsschnitt durch das Wirbelsäulenimplantat 100. Deutlich zu sehen sind die dem Innenraum IN zugewandten Flächen der Hintergriffselemente 154, 155 sowie die endseitig des Wirbelsäulenimplantates 100 vorhandenen Stützplatten 140. Die Stützplatten 140 können zur Anpassung der Lage des Wirbelsäulenimplantates 100 zwischen den benachbarten Wirbelkörpern geneigte Oberflächen aufweisen. Beispielsweise ist die am unteren Teil des in Fig. 6 gezeigten Wirbelsäulenimplantates vorhandene Fläche der Stützplatte 140 nicht senkrecht zur Mittellängsachse A orientiert. Zur rutschfesten Verriegelung des Wirbelsäulenimplantates 100 zwischen benachbarten Wirbelkörpern können endseitig an den Stützplatten 140 Dornen 160 vorhanden sein. Ebenfalls deutlich sichtbar ist der sich über die gesamte Länge des Wirbelsäulenimplantates 100 erstreckende Innenraum IN, der von den Enden des Implantates 100, d.h. im Bereich der Stützplatten 140 zugänglich ist.

Nach einem alternativen Ausführungsbeispiel weist das Wirbelsäulenimplantat 100 kein Halteelement 130 zur Stabilisierung der Freienden 201 des ersten Bauteils 101 auf (vgl. Fig. 2).

Ein bei Druckbelastung des Wirbelsäulenimplantates 100 auftretendes Spreizmoment der Wandsegmente 110, 111, 120, 121 wird dadurch abgefangen, dass das erste und zweite Bauteil 101, 102 mit einem in Radialrichtung wirksamen Formschluss miteinander verbunden sind. Ein radialer Formschluss kann vermittelt durch das Antriebselement 103 zwischen den Freienden 301 des zweiten Bauteils 102 hergestellt werden. Alternativ können die Wandsegmente 120, 121 des zweiten Bauteils 102 mit den Wandsegmenten 110, 111 des ersten Bauteils 101 einen radial wirksamen Formschluss eingehen.

Ein solcher radial wirksamer Formschluss zwischen dem ersten und zweiten Bauteil 101, 102, bzw. zwischen dem zweiten Bauteil 102 und dem Antriebselement 103, stellt jeweils für sich genommen eine Lösung zur Stabilisierung des Wirbelsäulenimplantates 100 dar, kann aber auch in Verbindung mit einem Halteelement 130 zum Einsatz gebracht werden.

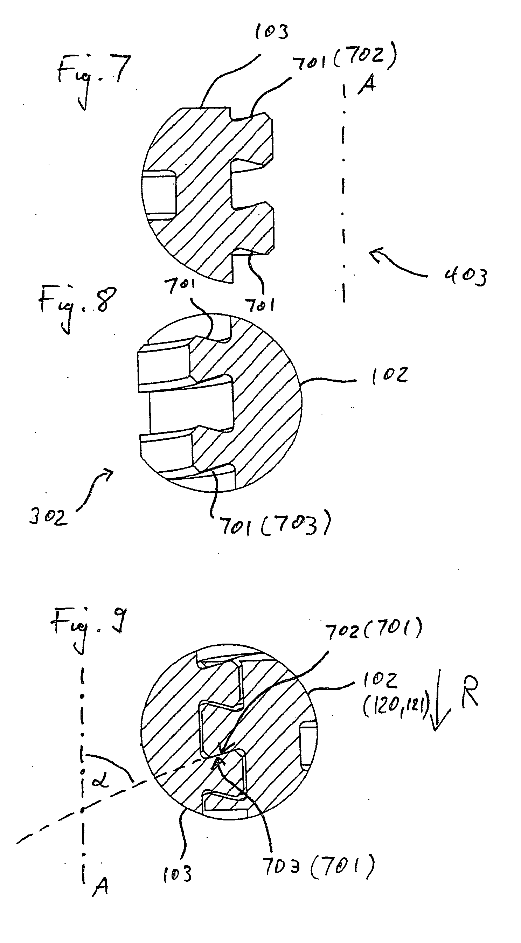

Gemäß der Ausführungsbeispiele in den Figuren 7 bis 9 weist das Antriebselement 103 ein im Querschnitt schwalbenschwanzförmig ausgestaltetes Außengewinde 403 auf. Das zweite Bauteil 102 weist ein entsprechend komplementär ausgestaltetes, ebenfalls schwalbenschwanzförmig ausgestaltetes Innengewinde 302 auf. Das schwalbenschwanzförmig ausgestaltete Außengewinde 403 des Antriebselementes 103 sowie das Innengewinde 302 des zweiten Bauteils 102 weisen gegenüber der Mittellängsachse A geneigte Flanken 701 auf. Zur Herstellung eines in Radialrichtung wirksamen Formschlusses, der bei einer Belastung des Wirbelsäulenimplantates 100 einem Spreizmoment der Freienden 301 der Wandsegmente 120, 121 des zweiten Bauteils 102 entgegen wirkt, ist entscheidend, dass die tragenden Flanken 702 des Antriebselementes 103 sowie die entsprechenden, ebenfalls tragenden Gegenflanken 703 des zweiten Bauelementes 102, gegenüber der Mittellängsachse A geneigt sind. So schließt die tragende Flanke 702 des Antriebselementes 103, wie Fig. 9 zeigt, mit der Mittellängsachse A einen in Richtung des zweiten Bauteils 102 geöffneten spitzen Winkel α ein. Die mit der tragenden Flanke 702 des Antriebselementes 103 zusammenwirkende Gegenflanke 703 des zweiten Bauteils 102 ist entsprechend komplementär ausgestaltet. Wirkt eine Kraft in Richtung der Lastrichtung R auf das zweite Bauteil 102, so wird auf Grund der geneigten Flanken 701 das entsprechende Wandsegment 120, 121 des zweiten Bauteils 102 ein Kraftmoment erfahren, welches in Richtung der Mittellängsachse A gerichtet ist. Dieses Kraftmoment wirkt einem auf die Wandsegmente 120, 121 des zweiten Bauteils 102 wirkenden Spreizmoment entgegen.

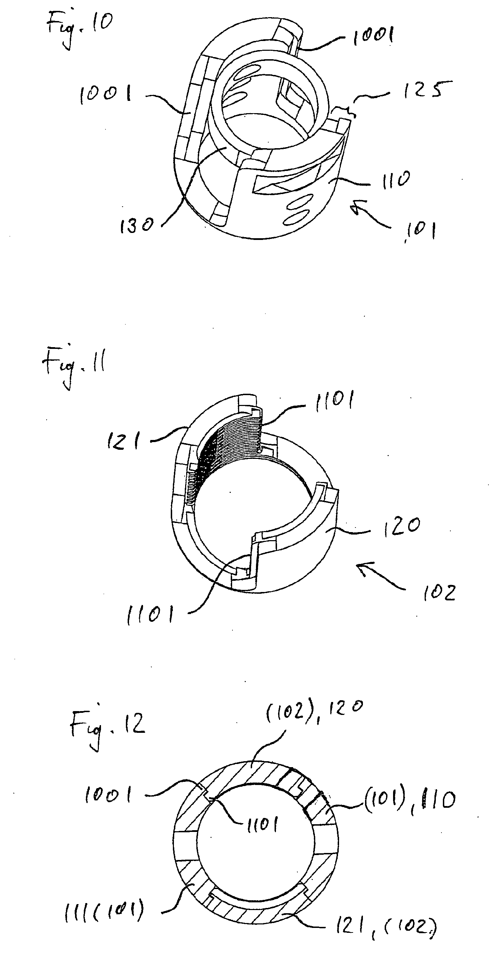

Alternativ oder gegebenenfalls zusätzlich zu einer schwalbenschwanzförmigen Ausgestaltung des Außengewindes 403 des Antriebselementes 103 und des Innengewindes 302 des zweiten Bauteils 102 ist nach einem weiteren Ausführungsbeispiel der in Radialrichtung wirksame Formschluss, wie die Figuren 10 bis 12 zeigen, zwischen den Wandsegmenten 110, 111 des ersten Bauteils 101 und den Wandsegmenten 120, 121 des zweiten Bauteils 102 durch an deren Schmalseiten 125 vorhandene Leisten 1001, 1101 gewährleistet. Fig. 10 zeigt eine Perspektivansicht des ersten Bauteils 101. Das erste Bauteil 101 weist an seinen Schmalseiten 125 in Umfangsrichtung vorspringende Leisten 1001 auf. Das in Fig. 11 gezeigte zweite Bauteil 102 weist entsprechend komplementär geformte Leisten 1101 im Bereich seiner Wandsegmente 120, 121 auf. Wie die Querschnittsansicht in Fig. 12 zeigt, hintergreifen die Leisten 1101 des zweiten Bauteils 102, in Radialrichtung gesehen, die Leisten 1001 des ersten Bauteils 101. Ein gegebenenfalls auf die Wandsegmente 120, 121 des zweiten Bauteils 102 wirkendes Spreizmoment kann, vermittelt durch die Leisten 1101, 1001, auf die Wandsegmente 110, 111 des ersten Bauteils 101 übertragen werden. Vorzugsweise erstrecken sich die Leisten 1001, 1101, in Axialrichtung gesehen, über die vollständige Länge der Schmalseiten 125 der Wandsegmente 110, 111, 120, 121. Das in Fig. 10 gezeigte erste Bauteil 101 weist wie auch das in Fig. 2 gezeigte Bauteil 101 ein Halteelement 130 auf, das die bereits beschriebenen Aufgaben und Funktionen erfüllt. Insbesondere wird ein von dem zweiten Bauteil 102 (vgl. Fig. 11), genauer dessen Wandsegmenten 120, 121 ausgehendes Spreizmoment, welches über die an dem ersten und zweiten Bauteil 101, 102 vorhandenen Leisten 1001, 1101 auf die Wandsegmente 110, 111 des ersten Bauteils 101 übertragen wird, von dem Halteelement 130 abgefangen.

Ein besonders bevorzugtes Material für ein medizinisches Implantat ist ein Werkstoff der auf der Basis von Titan, Edelstahl oder eines Polyetherketons (PEEK) hergestellt ist. Solche Materialien werden in der Regel von Körpergewebe nicht abgestoßen und verwachsen gut mit diesem. Außerdem weisen die vorstehenden Werkstoffe eine hohe Festigkeit auf. Zur Verbesserung seiner Festigkeit kann das PEEK-Material mit Kohlefasern verstärkt werden (CFK-PEEK).

## Patentansprüche

1. Höhenverstellbares Wirbelsäulenimplantat (100) mit
a) einem ersten und einem zweiten Bauteil (101, 102), die längs einer Mittellängsachse (A) des Implantats (100) gegeneinander drehfest und axial beweglich aneinander gehalten sind, mit
- jeweils zumindest zwei an einer Basis (112, 122) fixierten Wandsegmenten (110, 111, 120, 121), die sich in Richtung der Mittellängsachse (A) erstrecken und einen Radialabstand zu dieser aufweisen, wobei jeweils zwei in Umfangsrichtung benachbarte Wandsegmente (110, 111, 120, 121) einen Zwischenraum (S) flankieren, in den sich ein Wandsegment (110, 111, 120, 121) des anderen Bauteils erstreckt und darin axial geführt ist,
b) einem Antriebselement (103), welches
- in einem von den Wandsegmenten (110, 111, 120, 121) umschlossenen Innenraum (IN) des Implantats (100) angeordnet ist,
- mit dem zweiten Bauteil (102) nach der Art eines Schraubengetriebes zusammenwirkt,
- einen koaxial zur Mittellängsachse (A) angeordneten, zu seiner Drehbetätigung dienenden Zahnkranz (401) aufweist,
- sich in Lastrichtung (R) an dem ersten Bauteil (101) abstützt,
c) einer ein Wandsegment (110) des ersten Bauteils (101) durchsetzenden Zugriffsöffnung (150), über die der Zahnkranz (401) zur Drehbetätigung des Antriebselements (103) mit Hilfe eines Handhabungswerkzeugs zugänglich ist, **gekennzeichnet durch**
d) ein in dem Innenraum (IN) vorhandenes Halteelement (130), welches die Freienden (201) der Wandsegmente (110, 111) des ersten Bauteils (101) miteinander verbindet, wobei das Antriebselement (103) an dem Halteelement (130) um die Mittellängsachse (A) drehbar fixiert ist und sich in Lastrichtung (R) an einer Stützfläche (202) des Halteelements (130) abstützt.

2. Wirbelsäulenimplantat (100) nach Anspruch 1, **dadurch gekennzeichnet, dass** das Halteelement (130) und das erste Bauteil (101) einstückig ausgebildet sind.

3. Wirbelsäulenimplantat (100) nach Anspruch 2, **dadurch gekennzeichnet, dass** sich das Antriebselement (103) mit seinem Zahnkranz (401) auf der Stützfläche (202) abstützt.

4. Wirbelsäulenimplantat (100) nach Anspruch 2 oder 3, **dadurch gekennzeichnet, dass** der Zahnkranz (401) nach der Art eines Kronenrades eines Kronenradgetriebes ausgebildet ist, und das Halteelement (130) eine die Stützfläche (202) durchbrechende Ausnehmung aufweist, welche mit der Zugriffsöffnung (150) in Verbindung steht.

5. Wirbelsäulenimplantat (100) nach einem der Ansprüche 2 bis 4, **dadurch gekennzeichnet, dass** das im Halteelement (130) eine Durchgangsöffnung (203) aufweist, die von einem Fixierelement (501) durchgriffen ist, dessen eines Ende mit dem Antriebselement (103) verbunden ist, und dessen anderes Ende mit einem Hintergriffselement (502) das Halteelement (130) auf dessen dem Antriebselement (103) abgewandter Seite hintergreift.

6. Wirbelsäulenimplantat (100) nach einem der vorstehenden Ansprüche, **dadurch gekennzeichnet, dass** das Antriebselement (103) ein Außengewinde (403) aufweist, welches mit einem Innengewinde (302) des zweiten Bauteils (102) kämmt.

7. Wirbelsäulenimplantat (100) nach Anspruch 6, **dadurch gekennzeichnet, dass** das Innengewinde (302) in die Innenseiten der Wandsegmente (120, 121) des zweiten Bauteils (102) eingelassen ist.

8. Wirbelsäulenimplantat (100) nach Anspruch 6, **dadurch gekennzeichnet, dass** das Außengewinde (403) an dem dem zweiten Bauteil (102) zugewandten Ende des Antriebselement (103) angeordnet ist.

9. Wirbelsäulenimplantat (100) nach Anspruch 6, **dadurch gekennzeichnet, dass** das Antriebselement (103) derart an dem ersten Hülsenteil (101) angeordnet ist, dass das Außengewinde (403) - in Axialrichtung gesehen - sich außerhalb des ersten Hülsenteils (101) befindet.

10. Wirbelsäulenimplantat (100) nach einem der vorhergehenden Ansprüche, **dadurch gekennzeichnet, dass** an dem die Zugriffsöffnung (150) aufweisenden Wandsegment (110) mindestens ein der Radialfixierung eines Handhabungswerkzeugs dienendes Hintergriffselement (154, 155) vorhanden ist.

11. Wirbelsäulenimplantat (100) nach Anspruch 10, **dadurch gekennzeichnet, dass** das wenigstens eine Hintergriffselement (154, 155) eine in Richtung des Innenraums (IN) des Implantats (100) weisende Hintergriffsfläche aufweist.

12. Wirbelsäulenimplantat (100) nach einem der vorstehenden Ansprüche, **dadurch gekennzeichnet, dass** die Wandsegmente (110, 111, 120, 121) des ersten und zweiten Bauteils (101, 102) mit einem in Radialrichtung wirksamen Formschluss miteinander verbunden sind.

13. Wirbelsäulenimplantat (100) nach Anspruch 12, **dadurch gekennzeichnet, dass** die in Umfangsrichtung einander zugewandten Schmalseiten (125) der Wandsegmente (110, 111, 120, 121) zumindest auf einem Teil ihrer Länge formschlüssig ineinander greifen.

14. Wirbelsäulenimplantat (100) nach Anspruch 13, **dadurch gekennzeichnet, dass** die Wandsegmente an ihren Schmalseiten jeweils eine in Umfangsrichtung vorspringende Leiste (1001, 1101) aufweisen, wobei die Leisten (1001, 1101) des zweiten Bauteils (102) diejenigen des ersten Bauteils (101) hintergreifen.

15. Wirbelsäulenimplantat (100) nach einem der vorstehenden Ansprüche in Verbindung mit Anspruch 6, **dadurch gekennzeichnet, dass**
- die dem zweiten Bauteil (102) zugewandten, tragenden Flanken (702) des Außengewindes (403) des Antriebselementes (103) - in radialer Richtung betrachtet - gegenüber der Mittellängsachse (A) des Implantates (100) geneigt sind, und mit der Mittenlängsachse (A) einen zu dem zweiten Bauteil (102) geöffneten spitzen Winkel (α) einschließen und
- die mit den genannten Flanken (702) zusammenwirkenden Gegenflanken (703) des Innengewindes (302) des zweiten Bauteils (102) komplementär gestaltet sind.

16. Wirbelsäulenimplantat (100) nach Anspruch 15, **dadurch gekennzeichnet, dass** das Außengewinde (403) des Antriebselementes (103) und das Innengewinde (302) des zweiten Bauteils (102), in einer die Mittenlängsachse (A) enthaltenden Schnittebene betrachtet, schwalbenschwanzförmig ausgestaltet sind.

17. Wirbelsäulenimplantat (100) nach einem der vorstehenden Ansprüche, **dadurch gekennzeichnet, dass** an der zur Anlage an einen Wirbelkörper vorgesehenen Stirnseite eines Bauteils (101, 102) eine Stützplatte (140) vorhanden ist.

18. Wirbelsäulenimplantat (100) nach einem der vorstehenden Ansprüche, **dadurch gekennzeichnet, dass** zumindest Teile des Wirbelsäulenimplantats aus einem Werkstoff auf der Basis von Edelstahl, Titan oder einem Polyetherketon (PEEK) bestehen.

## Claims

1. Height-adjustable spinal implant (100) with
a) a first and a second component (101, 102), which are positioned along a middle longitudinal axis (A) of the implant (100) so that they cannot be rotated against each other but they can be moved axially towards each other, with
- each having at least two wall segments (110, 111, 120, 121) fixed onto a base (112, 122), the wall segments extending in the direction of the middle longitudinal axis (A) and having a radial distance from it, wherein in each case two wall segments (110, 111, 120, 121) - which are next to each other in the circumferential direction - flank a space (S) into which a wall segment (110, 111, 120, 121) of the other component extends and is axially led therein,
b) a drive element (103), which
- is located in an interior space (IN) enclosed by the wall segments (110, 111, 120, 121) of the implant (100),
- works together with the second component (102) in the manner of a screw gear,
- has a sprocket (401) located coaxial to the middle longitudinal axis (A) operating its rotation,
- supports itself on the first component (101) in the direction of load (R),
c) an access opening (150) going through a wall segment (110) of the first component (101), via which the sprocket (401) can be accessed with the help of a hand tool to rotate the drive element (103), **characterised by**
d) a retaining element (130) present in the interior space (IN), which joins together the free ends (201) of the wall segments (110, 111) of the first component (101), wherein the drive element (103) is fixed to the retaining element (130) so that it can be rotated around the middle longitudinal axis (A) and supports itself on a supporting surface (202) of the retaining element (130) in the direction of load (R).

2. Spinal implant (100) according to claim 1, **characterised in that** the retaining element (130) and the first component (101) are constructed in one piece.

3. Spinal implant (100) according to claim 2, **characterised in that** the drive element (103) with its sprocket (401) supports itself on the supporting surface (202).

4. Spinal implant (100) according to claim 2 or 3, **characterised in that** the sprocket (401) is constructed like the crown gear of a crown gearhead, and the retaining element (130) has a recess breaking through the supporting surface (202), a recess which is connected to the access opening (150).

5. Spinal implant (100) according to any one of the claims 2 to 4, **characterised in that** the retaining element (130) has a passage opening (203) in it that is gripped through by a fixing element (501), one end of which is connected to a drive element (103) and the other end of which grips back onto the side of the retaining element (130) facing away from the drive element (103) with a gripping-behind element (502).

6. Spinal implant (100) according to any one of the preceding claims, **characterised in that** the drive element (103) has an external thread (403), which threads into an internal thread (302) of the second component (102).

7. Spinal implant (100) according to claim 6, **characterised in that** the internal thread (302) is engaged into the interior side of the wall segments (120, 121) of the second component (102).

8. Spinal implant (100) according to claim 6, **characterised in that** the external thread (403) is located on the end of the drive element (103) facing towards the second component (102).

9. Spinal implant (100) according to claim 6, **characterised in that** the drive element (103) is located on the first casing part (101) in such a way that the external thread (403) - when looked at in the axial direction - is located outside the first casing part (101).

10. Spinal implant (100) according to any one of the preceding claims, **characterised in that** at least one gripping-behind element (154, 155) is present on the wall segment (110) that has the access opening (150), and serves to radially fix a hand tool.

11. Spinal implant (100) according to claim 10, **characterised in that** at least one gripping-behind element (154, 155) has a gripping-behind surface facing in the direction of the interior space (IN) of the implant (100).

12. Spinal implant (100) according to any one of the preceding claims, **characterised in that** the wall segments (110, 111, 120, 121) of the first and second components (101, 102) are joined to each other with a form closure acting in the radial direction.

13. Spinal implant (100) according to claim 12, **characterised in that** the narrow sides (125) of the wall segments (110, 111, 120, 121) that face each other in the circumferential direction interlock with each other on at least a part of their length.

14. Spinal implant (100) according to claim 13, **characterised in that** each of the wall segments has a trim (1001, 1101) on its narrow sides projecting in the circumferential direction, wherein the trims (1001, 1101) of the second component (102) grip behind on the those of the first component (101).

15. Spinal implant (100) according to any one of the preceding claims in connection with claim 6, **characterised in that**
- the load-bearing flanks (702) of the external thread (403) of the drive element (103) facing towards the second component (102) - looked at in the radial direction - are sloped opposite the middle longitudinal axis (A) of the implant (100), and comprise with the middle longitudinal axis (A) a vertex angle (α) to the second component (102) and
- mating flanks (703) working together with the mentioned flanks (702) of the internal thread (302) of the second component (102) are formed in a complementary fashion.

16. Spinal implant (100) according to claim 15, **characterised in that** the external thread (403) of the drive element (103) and the internal thread (302) of the second component (102), when looked at in one of the sectional planes containing the middle longitudinal axis (A), are formed in the shape of a dovetail.

17. Spinal implant (100) according to any one of the preceding claims, **characterised in that** a support plate (140) is present on a front side of a component (101, 102) which is provided as an appendix to a vertebral body.

18. Spinal implant (100) according to any one of the preceding claims, **characterised in that** at least parts of the spinal implant consist of a material based on stainless steel, titanium or polyether ether ketone (PEEK).

## Revendications

1. Implant de colonne vertébrale (100) réglable en hauteur, comprenant
a) une première et une deuxième pièces (101, 102) qui sont maintenues de manière à résister à la rotation l'une contre l'autre et mobiles axialement l'une sur l'autre le long d'un axe longitudinal médian (A) de l'implant (100), comprenant
- respectivement au moins deux segments de paroi (110, 111, 120, 121) fixés sur une base (112, 122) qui s'étendent en direction de l'axe longitudinal médian (A) et présentent un écart radial par rapport à celui-ci, sachant que respectivement deux segments de paroi (110, 111, 120, 121) adjacents dans le sens périphérique flanquent un espace intermédiaire (S) dans lequel s'étend un segment de paroi (110, 111, 120, 121) de l'autre pièce et qui est guidé axialement dedans,
b) un élément d'entraînement (103), lequel
- est agencé dans un espace intérieur (IN) de l'implant (100) entouré par les segments de paroi (110, 111, 120, 121),
- coopère avec la deuxième pièce (102) à la manière d'un système vis-écrou,
- présente une couronne dentée (104) disposée coaxialement à l'axe longitudinal médian (A) servant à effectuer sa rotation,
- s'appuie sur la première pièce (101) dans le sens de charge (R),
c) une ouverture d'accès (150) traversant un segment de paroi (110) de la première pièce (101) par laquelle la couronne dentée (104) est accessible à l'aide d'un outil de manipulation pour la rotation de l'élément d'entraînement (103), **caractérisé par**
d) un élément de fixation (130) présent dans l'espace intérieur (IN), lequel relie entre elles les extrémités libres (201) des segments de paroi (110, 111) de la première pièce (101), sachant que l'élément d'entraînement (103) est fixé sur l'élément de fixation (130) de manière à pouvoir tourner sur l'axe longitudinal médian (A) et s'appuie sur une surface d'appui (202) de l'élément de fixation (130) dans le sens de charge (R).

2. Implant de colonne vertébrale (100) selon la revendication 1, **caractérisé en ce que** l'élément de fixation (130) et la première pièce (101) sont formés de manière monobloc.

3. Implant de colonne vertébrale (100) selon la revendication 2, **caractérisé en ce que** l'élément d'entraînement (103) s'appuie sur la surface d'appui (202) avec sa couronne dentée (401).

4. Implant de colonne vertébrale (100) selon la revendication 2 ou 3, **caractérisé en ce que** la couronne dentée (401) est formée à la manière d'une roue dentée d'un engrenage à roue dentée et que l'élément de fixation (130) présente un évidement traversant la surface d'appui (202), lequel est en liaison avec l'ouverture d'accès (150).

5. Implant de colonne vertébrale (100) selon l'une des revendications 2 à 4, **caractérisé en ce que** l'élément de fixation (130) présente une ouverture traversante (203) qui est traversée par un élément de fixation (501) dont l'une extrémité est reliée à l'élément d'entraînement (103) et dont l'autre extrémité saisit par l'arrière, avec un élément d'engagement par l'arrière (502), l'élément de fixation (130) sur le côté détourné de l'élément d'entraînement (103) de celui-ci.

6. Implant de colonne vertébrale (100) selon l'une des revendications précédentes, **caractérisé en ce que** l'élément d'entraînement (103) est un filetage mâle (403) qui engrène la deuxième pièce (102) avec un filetage femelle (302).

7. Implant de colonne vertébrale (100) selon la revendication 6, **caractérisé en ce que** le filetage femelle (302) est intégré dans les côtés intérieurs des segments de paroi (120, 121) de la deuxième pièce (102).

8. Implant de colonne vertébrale (100) selon la revendication 6, **caractérisé en ce que** le filetage mâle (403) est disposé sur l'extrémité de l'élément d'entraînement (103) tournée vers la deuxième pièce (102).

9. Implant de colonne vertébrale (100) selon la revendication 6, **caractérisé en ce que** l'élément d'entraînement (103) est ainsi disposé sur la première partie de manchon (101), que le filetage mâle (403) - vu dans le sens axial - se trouve en-dehors de la première partie de manchon (101).

10. Implant de colonne vertébrale (100) selon l'une des revendications précédentes, **caractérisé en ce qu'**au moins un élément d'engagement par l'arrière (154, 155) servant à la fixation radiale d'un outil de manipulation, est présent sur le segment de paroi (110) présentant l'ouverture d'accès (150).

11. Implant de colonne vertébrale (100) selon la revendication 10, **caractérisé en ce que** l'au moins un élément d'engagement par l'arrière (154, 155) présente une surface d'engagement par l'arrière orientée en direction de l'espace intérieur (IN) de l'implant (100).

12. Implant de colonne vertébrale (100) selon l'une des revendications précédentes, **caractérisé en ce que** les segments de paroi (110, 111, 120, 121) des première et deuxième pièces (101, 102) sont reliés entre eux avec un procédé d'accouplement par complémentarité de forme agissant dans le sens radial.

13. Implant de colonne vertébrale (100) selon la revendication 12, **caractérisé en ce que** les côtés étroits (125) des segments de paroi (110, 111, 120, 121) tournés l'un vers l'autre dans le sens périphérique, se mettent en prise l'un dans l'autre par complémentarité de forme au moins sur une partie de leur longueur.

14. Implant de colonne vertébrale (100) selon la revendication 13, **caractérisé en ce que** les segments de paroi présentent sur leurs côtés étroits, respectivement une baguette (1001, 1101) faisant saillie dans le sens périphérique, sachant que les baguettes (1001, 1101) de la deuxième pièce engagent par l'arrière celles de la première pièce (101).

15. Implant de colonne vertébrale (100) selon l'une des revendications précédentes en liaison avec la revendication 6, **caractérisé en ce que**
- les flancs porteurs (702) du filetage mâle (403) de l'élément d'entraînement (103), tournés vers la deuxième pièce (102) - considéré dans le sens radial - sont inclinés par rapport à l'axe longitudinal médian (A) de l'implant (100) et, avec l'axe longitudinal médian (A), incluent un angle aigu (α) ouvert vers la deuxième pièce (102) et
- les contre-flancs (703) du filetage femelle (302) de la deuxième pièce (102) coopérant avec lesdits flancs (702), sont formés de manière complémentaire.

16. Implant de colonne vertébrale (100) selon la revendication 15, **caractérisé en ce que** le filetage mâle (403) de l'élément d'entraînement (103) et le filetage femelle (302) de la deuxième pièce (102), considérés dans un plan de coupe contenant l'axe longitudinal médian (A), sont en forme de queue d'aronde.

17. Implant de colonne vertébrale (100) selon l'une des revendications précédentes, **caractérisé en ce qu'**un plateau d'appui (140) est présent sur le côté avant d'une pièce (101, 102) prévu pour venir se poser sur un corps vertébral.

18. Implant de colonne vertébrale (100) selon l'une des revendications précédentes, **caractérisé en ce qu'**au moins des parties de l'implant de colonne vertébrale sont composées d'un matériau à base d'acier inoxydable, de titane ou d'un polyétheréthercétone (PEEK).
